# EUROPEAN PATENT APPLICATION

(11) **EP 4 599 872 A1**
(43) Date of publication of application: **13.08.2025**
(21) Application number: 24837521.4
(22) Date of filing: 04.11.2024
(51) Int. Cl.: A61M 11/02

(54) **ATOMIZER**

(30) Priority: 27.12.2023 CN 202311822841
(71) Applicant: Shenzhen AOJ Health Technology Co., Ltd., Shenzhen, Guangdong 518126 (CN)
(72) Inventor: GAO, Yongjie, Shenzhen, Guangdong 518126 (CN)
(74) Representative: Cleanthous, Marinos
(86) International application number: PCT/CN2024/129768
(87) International publication number: WO 2025/139371

(57) **Abstract**

Disclosed is a nebulizer, comprising: an nebulization assembly; an automatic feeding assembly connected to the nebulization assembly for automatically adding liquid medicine into the nebulization assembly; a power assembly configured to deliver compressed air to the nebulization assembly that nebulizes the liquid medicine using the delivered compressed air; a flow divider assembly disposed between the power assembly and the nebulization assembly for diverting a part of the delivered airflow; and an adjusting member configured to adjust a proportion of the diverted airflow so as to regulate sizes of nebulized particles produced by the nebulization assembly. Since the adjusting member is capable of adjusting the flow distribution ratio of the flow divider assembly, it can produce a medicinal mist tailored to specific particle size characteristics to cater to the medication needs of different users, resulting in a simple product structure and low production costs.

## Description

### FIELD

The present disclosure relates to medical devices, particularly to nebulizers.

### BACKGROUND

Compressed nebulizers are commonly used medical devices in respiratory therapy, mainly classified into direct current powered nebulizers and alternating current powered nebulizers. Both can atomize liquid medicine through the Venturi injection principle, turning the liquid medicine into extremely fine nebulized particles for inhalation by human, thereby achieving the effect of painless and rapid treatment.

In existing nebulizers, the pressure and flow provided by the power structure affect the deposition site of the drug within the human body (i.e., the acting site of the drug). In related technologies, most nebulizers with adjustable pressure or flow have complex structures, high production costs and poor stability of nebulized particles, making it difficult to meet the needs of users.

### SUMMARY

In view of the above issues with existing nebulizers, the present disclosure aims to provide nebulizers which are simple in structure, low in cost and high in stability.

The specific technical solutions are as follows.

A nebulizer comprises: an nebulization assembly; an automatic feeding assembly connected to the nebulization assembly for automatically adding liquid medicine into the nebulization assembly; a power assembly configured to deliver compressed air to the nebulization assembly that nebulizes the liquid medicine using the compressed air delivered by the power assembly; a flow divider assembly disposed between the power assembly and the nebulization assembly for diverting a part of the airflow delivered from the power assembly to the nebulization assembly; and an adjusting member configured to adjust a proportion of the airflow diverted by the flow divider assembly so as to regulate sizes of nebulized particles of the liquid medicine produced by the nebulization assembly.

As a further enhancement and optimization to this solution, the nebulization assembly may include: an airflow channel with an opening at its bottom, the opening communicating with the power assembly; a medicine cup disposed on both sides of the airflow channel and enclosed at both its upper and lower ends, the medicine cup having a liquid inlet on its top side wall, a medicine chamber for storing the liquid medicine being formed inside the medicine cup between the liquid inlet and a bottom of the medicine cup, and the automatic feeding assembly being in communication with the liquid inlet for automatically delivering the liquid medicine into the medicine chamber; and an impact baffle disposed directly above the airflow channel, with both sides of the airflow channel equipped with liquid suction tubes, one end of each liquid suction tube being inserted into the medicine cup and extended to a bottom of the medicine chamber, and the other end of each liquid suction tube facing the impact baffle.

As a further enhancement and optimization to this solution, the automatic feeding assembly may include: a liquid supply tube, with one end communicated with the liquid inlet via a connecting tube, and the other end provided with a plurality of ventilation holes; a sliding piston movably disposed inside the liquid supply tube and forming a sealed sliding engagement with an inner wall of the liquid supply tube; and a pull rod, with one end connected to a side of the sliding piston that is away from the connecting tube, and the other end extended to an exterior of the liquid supply tube.

As a further enhancement and optimization to this solution, the communicating tube may be a flexible tube.

As a further enhancement and optimization to this solution, the nebulizer may further include a housing, wherein the nebulization assembly, the flow divider assembly and the power assembly are all disposed inside the housing, the housing is provided with an exhaust hole that is in communication with the flow divider assembly, and the adjusting member is disposed at the exhaust hole for opening or sealing the exhaust hole.

As a further enhancement and optimization to this solution, the housing may be provided with a mounting groove, the exhaust hole may be eccentrically disposed at a bottom of the mounting groove; and the adjusting member may comprise a rotating part that is coaxially and rotatably disposed within the mounting groove, and a side of the rotating part facing the mounting groove is eccentrically provided with a sealing part that is in a sealing engagement with the exhaust hole.

As a further enhancement and optimization to this solution, at least a set of limiting assembly may be provided between the rotating part and the mounting groove, the limiting assembly may comprise a limiting protrusion disposed on an outer wall of the rotating part and a limiting slot disposed on an inner wall of the mounting groove, the limiting protrusion may be engaged within the limiting slot and forms a circumferential sliding fit therewith, when the limiting protrusion is circumferentially limited by a side wall of the limiting slot, the sealing part may be in a sealing engagement with the exhaust hole, and when the limiting protrusion is circumferentially limited by another side wall of the limiting slot, the sealing part may be in a misaligned engagement with the exhaust hole to open the exhaust hole.

As a further enhancement and optimization to this solution, a notch may be provided on an outer circumferential surface of the rotating part, and the limiting protrusion may be disposed at the notch.

As a further enhancement and optimization to this solution, an annular groove may be provided on an outer circumference of the rotating part, an annular flange may be disposed in an annular manner on a wall of the mounting groove and may be coaxially arranged inside the annular groove to form a circumferential guiding engagement with the annular groove, and when the annular flange is inside the annular groove, the sealing part may be in contact with the bottom of the mounting groove.

As a further enhancement and optimization to this solution, the flow divider assembly may comprise a three-way valve that includes an inlet end communicating with the power assembly, an outlet end communicating with the nebulization assembly, and an exhaust end communicating with the exhaust hole, and a uniform leak valve may be provided between the exhaust end and the exhaust hole.

The flow divider assembly may further include a sealing tube and a connecting tube, the connecting tube may be connected between the uniform leak valve and the exhaust hole, the sealing tube may be disposed in a sealing manner around the exteriors of the exhaust end, the connecting tube and the uniform leak valve. The sealing tube may be a plastic tube.

The positive effects of the aforementioned technical solution compared to the prior art may be as follows:
(1) The adjusting member in the present disclosure can regulate the flow distribution ratio of the flow divider assembly, thereby enabling flexible control of the air pressure and airflow reaching the nebulization assembly. This allows the atomization of medicinal mist with the desired particle size characteristics to cater to the medication needs of different users, resulting in a simple product structure and low production costs.
(2) The automatic feeding assembly in the present disclosure is designed to function as follows. When the amount of liquid medicine in the medicine cup is reduced due to being drawn outwards, leading to a relative decrease in the liquid level, the air pressure inside the medicine cup drops and becomes lower than the external air pressure. Driven by the external atmospheric pressure, the sliding piston is automatically pushed inward, squeezing the liquid medicine from the liquid supply tube into the medicine cup. This not only ensures the amount of the liquid medicine in the medicine cup to meet the requirement for sustained nebulization, but also maintains a relatively stable hydraulic pressure within the medicine cup. In this way, it prevents fluctuations in negative pressure at the top of the liquid suction tube caused by changes in hydraulic pressure due to a decrease in the amount of liquid medicine, which could otherwise affect the size of the nebulized particles. As a result, the stability of the atomization of the liquid medicine is improved.
(3) To facilitate users to adjust the size of the nebulized particles of the liquid medicine more precisely, when the limiting protrusion is circumferentially limited by a side wall of the limiting slot, the sealing part is in a sealing engagement with the exhaust hole; and when the limiting protrusion is circumferentially limited by the other side wall of the limiting slot, the sealing part is in a misaligned fit with the exhaust hole to open the exhaust hole.

### BRIEF DESCRIPTION OF THE DRAWINGS

FIG. 1 is a schematic diagram of the overall structure of a nebulizer according to the present disclosure;
FIG. 2 is a schematic diagram of the structure of the nebulization assembly of a nebulizer according to the present disclosure;
FIG. 3 is a schematic diagram of the structure of the automatic feeding assembly of a nebulizer according to the present disclosure;
FIG. 4 is a schematic diagram of the structure of the housing of a nebulizer according to the present disclosure;
FIG. 5 is a schematic diagram of the structure of the adjusting member of a nebulizer according to the present disclosure; and
FIG. 6 is a schematic diagram of the connection between the adjusting member and the flow divider assembly of a nebulizer according to the present disclosure.

### List of Reference Numerals

- 1: power assembly
- 2: nebulization assembly
- 3: flow divider assembly
- 4: adjusting member
- 5: housing
- 21: impact baffle
- 22: airflow channel
- 23: medicine cup
- 24: liquid suction tube
- 231: liquid inlet
- 31: three-way valve
- 32: uniform leak valve
- 33: connecting tube
- 34: sealing tube
- 41: rotating part
- 42: sealing part
- 411: limiting protrusion
- 412: annular groove
- 51: mounting groove
- 52: exhaust hole
- 53: annular flange
- 511: limiting slot
- 61: liquid supply tube
- 62: communicating tube
- 63: sliding piston
- 64: pull rod
- 631: ventilation hole

### DETAILED DESCRIPTION

The following further illustrates the present disclosure with reference to the accompanying drawings and specific embodiments, but it shall not be construed as a limitation to the present disclosure.

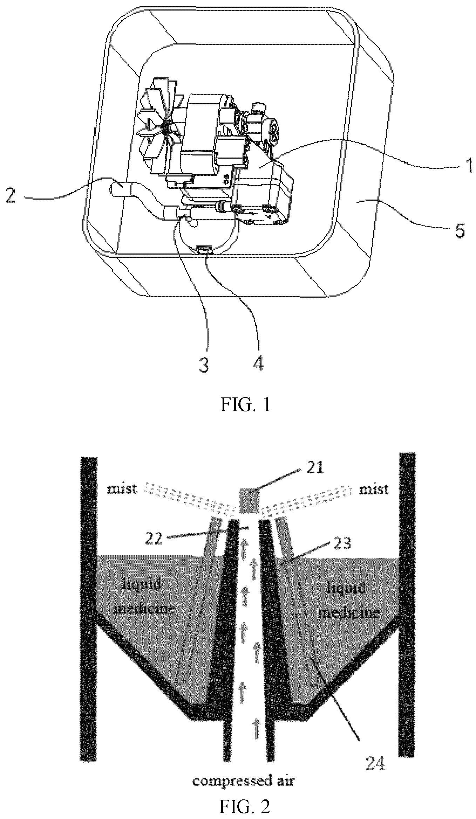

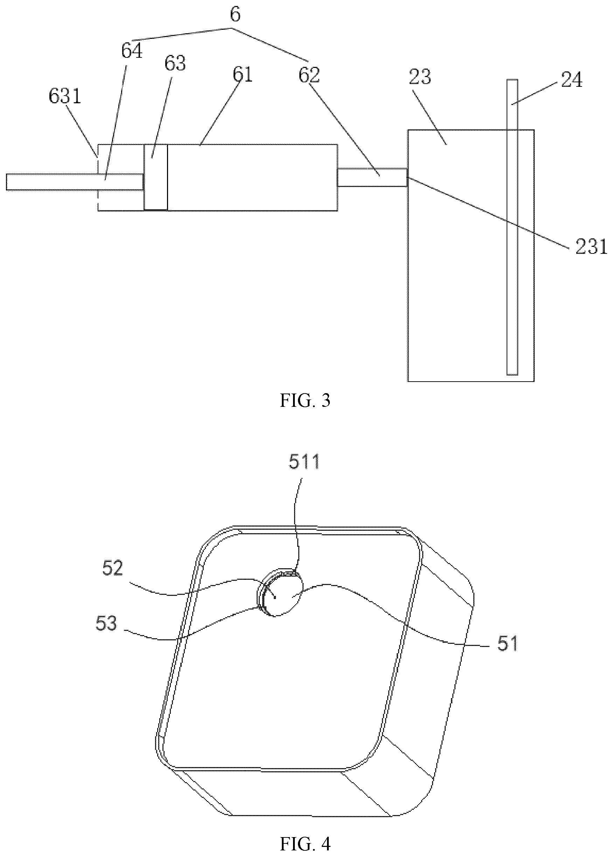

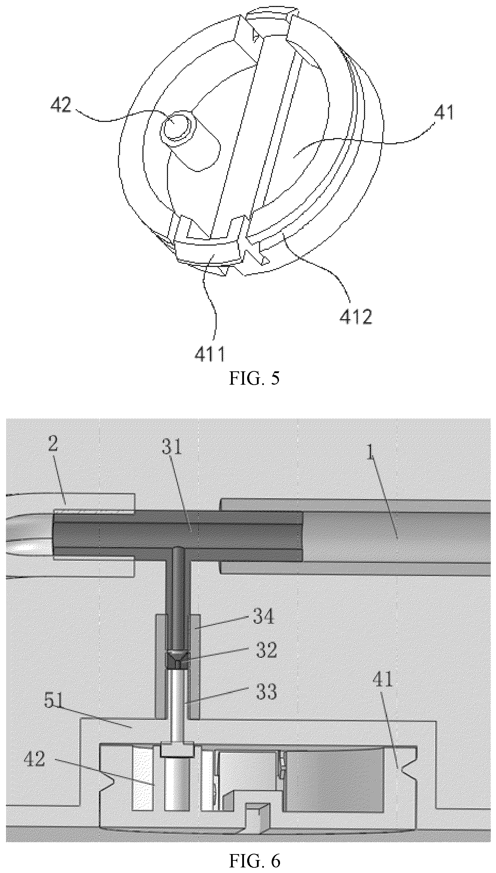

FIG. 1 schematically shows the overall structure of a nebulizer according to the present disclosure, FIG. 2 schematically shows the structure of the nebulization assembly of the nebulizer, FIG. 3 schematically shows the structure of the automatic feeding assembly of the nebulizer, FIG. 4 schematically shows the structure of the housing of the nebulizer, FIG. 5 schematically shows the structure of the adjusting member of the nebulizer, and FIG. 6 schematically shows the connection between the adjusting member and the flow divider assembly of the nebulizer. As shown in FIGS. 1 to 6, a nebulizer shown in some preferred embodiments may include: a power assembly 1, an automatic feeding assembly, a nebulization assembly 2, a flow divider assembly 3 and an adjusting member 4. The automatic feeding assembly may be connected to the nebulization assembly 2 for automatically adding liquid medicine into the nebulization assembly 2. The power assembly 1 may be configured to deliver compressed air to the nebulization assembly 2 which may nebulize the liquid medicine using the the compressed air delivered by the power assembly 1. The flow divider assembly 3 may be disposed between the power assembly 1 and the nebulization assembly 2 for diverting a part of the airflow delivered from the power assembly 1 to the nebulization assembly 2. The adjusting member 4 may be configured to adjust the proportion of the airflow diverted by the flow divider assembly 3 so as to regulate the size of the nebulized particles produced by the nebulization assembly 2.

In the shown embodiments, since the adjusting member 4 is capable of adjusting the flow distribution ratio of the flow divider assembly 3, it becomes possible to flexibly control the air pressure and airflow reaching the nebulization assembly 2. Consequently, it can produce a medicinal mist tailored to specific particle size characteristics to cater to the medication needs of different users. Moreover, this design leads to a simple product structure and low production costs.

In some preferred embodiments, the power assembly 1 may be a power pump, specifically a piston pump.

Furthermore, in some preferred embodiments, the nebulization assembly 2 may include an airflow channel 22, a medicine cup 23 and an impact baffle 21. The bottom of the airflow channel 22 has an opening, through which it can be in communication with the power assembly 1. The medicine cup 23 is arranged in such a way that it flanks both sides of the airflow channel 22 and is enclosed at both the upper and lower ends. The top side wall of the medicine cup 23 has a liquid inlet 231. A medicine chamber for storing liquid medicine is formed inside the medicine cup 23 between the liquid inlet 231 and the bottom of the medicine cup 23. The automatic feeding assembly is in communication with the liquid inlet 231 for automatically delivering liquid medicine into the medicine chamber. The impact baffle 21 is disposed directly above the airflow channel 22, and both sides of the airflow channel 22 are equipped with liquid suction tubes 24. One end of each liquid suction tube 24 extends into the medicine cup 23 and reaches the bottom of the medicine chamber, while the other ends faces the impact baffle 21.

In such embodiments, when the power assembly 1 supplies compressed air at a certain pressure to the airflow channel 22, a negative pressure is formed at the top of the liquid suction tube 24. Subsequently, the liquid medicine within the medicine cup 23 is drawn out and sprayed towards the impact baffle 21, thus forming fine nebulized particles that are then ejected out for users to inhale.

Furthermore, in some preferred embodiments, the automatic feeding assembly may include a liquid supply tube 61, a sliding piston 63 and a pull rod 64. One end of the liquid supply tube 61 is in communication with the liquid inlet 231 via a communicating tube 62. The sliding piston 63 is movably disposed inside the liquid supply tube 61 and forms a sealed sliding fit with the inner wall of the liquid supply tube 61. One end of the pull rod 64 is connected to the side of the sliding piston 63 that is away from a connecting tube 33, and the other end extends to the exterior of the liquid supply tube 61 via the other end of the liquid supply tube 61. The other end of the liquid supply tube 61 is provided with a plurality of ventilation holes 631.

In such embodiments, the automatic feeding assembly is designed to function as follows. When the amount of liquid medicine in the medicine cup 23 is reduced due to being drawn outwards, leading to a relative decrease in the liquid level, the air pressure inside the medicine cup 23 drops and becomes lower than the external air pressure. Driven by the external atmospheric pressure, the sliding piston 63 is automatically pushed inward, squeezing the liquid medicine from the liquid supply tube 61 into the medicine cup 23. This not only ensures the amount of the liquid medicine in the medicine cup 23 to meet the requirement for sustained nebulization, but also maintains a relatively stable hydraulic pressure within the medicine cup 23. In this way, it prevents fluctuations in negative pressure at the top of the liquid suction tube 24 caused by changes in hydraulic pressure due to a decrease in the amount of liquid medicine, which could otherwise affect the size of the nebulized particles. As a result, the stability of the atomization of the liquid medicine is improved.

Furthermore, in some preferred embodiments, the communicating tube 62 may be a flexible tube, so as to expand the installation scenarios for the liquid supply tube 61.

Furthermore, in some preferred embodiments, the nebulizer may further include a housing 5, within which the nebulization assembly 2, the flow divider assembly 3 and the power assembly 1 are all disposed. The housing 5 is provided with an exhaust hole 52 that is in communication with the flow divider assembly 3. The adjusting member 4 is disposed at the exhaust hole 52 for opening or sealing the exhaust hole 52.

Furthermore, in some preferred embodiments, the housing 5 is provided with a mounting groove 51, and the exhaust hole 52 is eccentrically disposed at the bottom of the mounting groove 51. The adjusting member 4 may include a rotating part 41 that is coaxially and rotatably disposed within the mounting groove 51. The side of the rotating part 41 facing the mounting groove 51 is eccentrically provided with a sealing part 42. The sealing part 42 is in a sealing engagement with the exhaust hole 52. In this way, users can rotate the rotating part 41 to control to open the sealing part 42 or seal the exhaust hole 52, thereby achieving the regulation of flow distribution.

Furthermore, the rotating part 41 and the sealing part 42 may be either integrated as a single structure or a detachable structure.

Furthermore, in some preferred embodiments, at least a set of limiting assembly is provided between the rotating part 41 and the mounting groove 51. The limiting assembly may include a limiting protrusion 411 disposed on the outer wall of the rotating part 41 and a limiting slot 511 disposed on the inner wall of the mounting groove 51. The limiting protrusion 411 is engaged within the limiting slot 511 and forms a circumferential sliding fit therewith. Moreover, when the limiting protrusion 411 is circumferentially limited by a side wall of the limiting slot 511, the sealing part 42 is in a sealing engagement with the exhaust hole 52; and when the limiting protrusion 411 is circumferentially limited by another side wall of the limiting slot 511, the sealing part 42 is in a misaligned fit with the exhaust hole 52 to open the exhaust hole 52.

In such embodiments, to facilitate users to adjust the size of the nebulized particles of the liquid medicine more precisely, when the limiting protrusion 411 is circumferentially limited by a side wall of the limiting slot 511, the sealing part 42 is in a sealing engagement with the exhaust hole 52; and when the limiting protrusion 411 is circumferentially limited by the other side wall of the limiting slot 511, the sealing part 42 is in a misaligned fit with the exhaust hole 52 to open the exhaust hole 52.

Furthermore, in some preferred embodiments, to facilitate the installation of the limiting protrusion 411, a notch is provided on the outer circumferential surface of the rotating part 41. The limiting protrusion 411 is disposed at the notch, allowing it to deform during installation, and then facilitating easy installation into the limiting slot 511.

Furthermore, in some preferred embodiments, to improve the installation stability of the rotating part 41, an annular groove 412 is provided on the outer circumference of the rotating part 41. An annular flange 53 is disposed in an annular manner on the wall of the mounting groove 51 and is coaxially arranged inside the annular groove 412 to form a circumferential guiding engagement with the annular groove 412. When the annular flange 53 is inside the annular groove 412, the sealing part 42 is in contact with the bottom of the mounting groove 51.

Furthermore, in some preferred embodiments, the flow divider assembly 3 may include a three-way valve 31 that may include an inlet end communicating with the power assembly 1, an outlet end communicating with the nebulization assembly 2, and an exhaust end communicating with the exhaust hole 52. A uniform leak valve 32, including a constant-rate leak valve, is provided between the exhaust end and the exhaust hole 52.

In such embodiments, the uniform leak valve 32 is designed to enable a more stable shunting process.

Additionally, the flow divider assembly 3 may further include a sealing tube 34 and the connecting tube 33. The connecting tube 33 is connected between the uniform leak valve and the exhaust hole 52. The sealing tube 34 is disposed in a sealing manner around the exteriors of the exhaust end, the connecting tube 33 and the uniform leak valve 32. The sealing tube 34 may be a plastic tube, which is beneficial for enhancing the sealing performance and connection stability of the exteriors of the exhaust end, the connecting tube 33 and the uniform leak valve 32.

The above description is merely preferred embodiments of the present disclosure and is not intended to limit the implementation modes and scope of protection of the present disclosure. For those skilled in the art, it should be readily apparent that any equivalent substitutions and obvious variations made based on the specification and drawings of the present disclosure shall fall within the scope of protection of the present disclosure.

## Claims

1. A nebulizer, comprising:
an nebulization assembly;
an automatic feeding assembly connected to the nebulization assembly for automatically adding liquid medicine into the nebulization assembly;
a power assembly configured to deliver compressed air to the nebulization assembly that nebulizes the liquid medicine using the compressed air delivered by the power assembly;
a flow divider assembly disposed between the power assembly and the nebulization assembly for diverting a part of the airflow delivered from the power assembly to the nebulization assembly; and
an adjusting member configured to adjust a proportion of the airflow diverted by the flow divider assembly so as to regulate sizes of nebulized particles of the liquid medicine produced by the nebulization assembly.

2. The nebulizer according to claim 1, wherein the nebulization assembly comprises:
an airflow channel with an opening at its bottom, the opening communicating with the power assembly;
a medicine cup disposed on both sides of the airflow channel and enclosed at both its upper and lower ends, the medicine cup having a liquid inlet on its top side wall, a medicine chamber for storing the liquid medicine being formed inside the medicine cup between the liquid inlet and a bottom of the medicine cup, and the automatic feeding assembly being in communication with the liquid inlet for automatically delivering the liquid medicine into the medicine chamber; and
an impact baffle disposed directly above the airflow channel, with both sides of the airflow channel equipped with liquid suction tubes, one end of each liquid suction tube being inserted into the medicine cup and extended to a bottom of the medicine chamber, and the other end of each liquid suction tube facing the impact baffle.

3. The nebulizer according to claim 2, wherein the automatic feeding assembly comprises:
a liquid supply tube, with one end communicated with the liquid inlet via a connecting tube, and the other end provided with a plurality of ventilation holes;
a sliding piston movably disposed inside the liquid supply tube and forming a sealed sliding engagement with an inner wall of the liquid supply tube; and
a pull rod, with one end connected to a side of the sliding piston that is away from the connecting tube, and the other end extended to an exterior of the liquid supply tube.

4. The nebulizer according to claim 3, wherein the connecting tube is a flexible tube.

5. The nebulizer according to claim 1, further comprising a housing, wherein the nebulization assembly, the flow divider assembly and the power assembly are all disposed inside the housing, the housing is provided with an exhaust hole that is in communication with the flow divider assembly, and the adjusting member is disposed at the exhaust hole for opening or sealing the exhaust hole.

6. The nebulizer according to claim 5, wherein the housing is provided with a mounting groove, the exhaust hole is eccentrically disposed at a bottom of the mounting groove; and
the adjusting member comprises a rotating part that is coaxially and rotatably disposed within the mounting groove, and a side of the rotating part facing the mounting groove is eccentrically provided with a sealing part that is in a sealing engagement with the exhaust hole.

7. The nebulizer according to claim 6, wherein at least a set of limiting assembly is provided between the rotating part and the mounting groove, the limiting assembly comprises a limiting protrusion disposed on an outer wall of the rotating part and a limiting slot disposed on an inner wall of the mounting groove, the limiting protrusion is engaged within the limiting slot and forms a circumferential sliding fit therewith, when the limiting protrusion is circumferentially limited by a side wall of the limiting slot, the sealing part is in a sealing engagement with the exhaust hole, and when the limiting protrusion is circumferentially limited by another side wall of the limiting slot, the sealing part is in a misaligned engagement with the exhaust hole to open the exhaust hole.

8. The nebulizer according to claim 7, wherein a notch is provided on an outer circumferential surface of the rotating part, and the limiting protrusion is disposed at the notch.

9. The nebulizer according to claim 6, wherein an annular groove is provided on an outer circumference of the rotating part, an annular flange is disposed in an annular manner on a wall of the mounting groove and is coaxially arranged inside the annular groove to form a circumferential guiding engagement with the annular groove, and when the annular flange is inside the annular groove, the sealing part is in contact with the bottom of the mounting groove.

10. The nebulizer according to claim 5, wherein the flow divider assembly comprises a three-way valve that includes an inlet end communicating with the power assembly, an outlet end communicating with the nebulization assembly, and an exhaust end communicating with the exhaust hole, and a uniform leak valve is provided between the exhaust end and the exhaust hole.
